# EUROPEAN PATENT SPECIFICATION

(11) **EP 3 939 392 B1**
(45) Date of publication and mention of the grant of the patent: **06.05.2026**
(21) Application number: 20713271.3
(22) Date of filing: 19.03.2020
(51) Int. Cl.: H05H 9/04

(54) **METHOD OF DETERMINING WHETHER REPAIR OR REPLACEMENT OF A MAGNETRON OF A RADIOTHERAPY LINEAR ACCELERATOR SHOULD BE SCHEDULED, AND COMPUTER READABLE MEDIUM TO PERFORM THE METHOD**
VERFAHREN ZUR BESTIMMUNG, OB EINE REPARATUR ODER EIN AUSTAUSCH EINES MAGNETRONS EINES LINEARBESCHLEUNIGERS FÜR DIE STRAHLENTHERAPIE GEPLANT WERDEN SOLLTE, UND COMPUTERLESBARES MEDIUM ZUR DURCHFÜHRUNG DES VERFAHRENS
MÉTHODE PERMETTANT DE DÉTERMINER SI LA RÉPARATION OU LE REMPLACEMENT D'UN MAGNÉTRON D'UN ACCÉLÉRATEUR LINÉAIRE POUR RADIOTHÉRAPIE DOIT ÊTRE PLANIFIÉ, ET SUPPORT LISIBLE PAR ORDINATEUR POUR METTRE EN OEUVRE LA MÉTHODE

(30) Priority: 20.03.2019 GB 201903820
(43) Date of publication of application: 19.01.2022
(73) Proprietor: Elekta Limited, Crawley, West Sussex RH10 9BL (GB)
(72) Inventor: CHIAP, Alessandra, London Road Crawley RH10 9RR (GB); VEGA, German, London Road Crawley RH10 9RR (GB); RICHARDSON, Keith, London Road Crawley RH10 9RR (GB); REED, Stuart, London Road Crawley RH10 9RR (GB); FLINT, Chris, London Road Crawley RH10 9RR (GB); JORDAO, Marcelo, London Road Crawley RH10 9RR (GB)
(74) Representative: Harden, Henry Simon
(86) International application number: PCT/EP2020/057706
(87) International publication number: WO 2020/188065

(56) References cited:
- US-A- 2 913 619
- US-A1- 2003 072 411
- US-A1- 2013 085 702
- LEE SEUNG HYUN ET AL: "X-band Linac for a 6MeV dual-head radiation therapy gantry", NUCLEAR INSTRUMENTS & METHODS IN PHYSICS RESEARCH. SECTION A, vol. 852, 8 February 2017 (2017-02-08), pages 40 - 45, XP029939905, ISSN: 0168-9002, DOI: 10.1016/J.NIMA.2016.11.034

## Description

### FIELD

The present disclosure relates to a radiotherapy device, and to a method of monitoring a radiotherapy device.

### BACKGROUND

Radiotherapy is an important tool in modern cancer treatment. Radiotherapy devices are large, complex machines, with many moving parts and inter-operating mechanisms. Despite precision engineering and rigorous testing, some component parts of a radiotherapy device may start to degrade over its lifetime. This can sometimes lead to sub-optimal operation and even the occasional safety override.

If at any point during treatment a radiotherapy device starts to function outside of its normal operating parameters, a safety override or "interrupt" occurs, whereby the machine stops delivering radiation to ensure patient safety. Such an event is inconvenient, as it adds time to the treatment, and in some cases means the treatment session must finish prematurely. Unplanned equipment downtime can disrupt planned treatment schedules, and may be expensive for the machine owner, be it due to loss of revenue, servicing and repair costs, or both.

It has been surmised that predictive maintenance and/or remote diagnostic techniques could be applied to radiotherapy machines. Identifying the link between particular data patterns and the particular fault or degrading component is often non-intuitive even for experienced service engineers.

The present disclosure relates generally to collecting data from a radiotherapy device, particularly to collecting and remotely analysing data from a of radiotherapy device, and using analysis to determine if a magnetron of a radiotherapy device is nearing the time at which it should be replaced or repaired. To date, no such predictive approach has been possible, and existing methods of servicing and repair of a radiotherapy device require sending a field service engineer to inspect the machine, take measurements from the magnetron, and diagnose and fix the problem.

Since the type of problem or the component which is at fault is not known in advance, time consuming diagnostic testing must be performed on-site. Existing methods therefore result in a significant amount of machine down time. Also, in existing methods, a field service engineer is not made aware of a potential issue until the component has degraded to a point where the radiotherapy machine is undergoing safety interrupts, or even until the point where the radiotherapy machine cannot operate within its safety parameters. This means that the servicing of the radiotherapy machine is often scheduled at a time which is inconvenient, or inefficient in terms of both field service engineer resources and the resources of the hospital or other machine owner. The present invention seeks to address these and other disadvantages encountered in the prior art by providing a method of determining, preferably remotely, whether repair or replacement of a magnetron in a radiotherapy device should be scheduled.
US2003/072411 A1 and
Lee Sung Hyun et al."X-band Linac for a 6 MeV dual-head radiation therapy gantry" NIM A, vol.852, pp.40-45
are examples of radiotherapy linear accelerators powered by a magnetron.

### SUMMARY

In a first aspect the invention defines a method of determining whether repair or replacement of a magnetron of a linear accelerator should be scheduled, according to claim 1.

In a second aspect the invention defines a computer readable medium according to claim 7.

Further advantageous embodiments are defined in the dependent claims.

### BRIEF DESCRIPTION OF THE DRAWINGS

Specific embodiments are described below by way of example only and with reference to the accompanying drawings in which:
Figure 1 illustrates a radiotherapy device according to an aspect of the disclosure;
Figure 2 illustrates a block diagram of a radiotherapy device according to the disclosure;
Figure 3 illustrates a method of predicting a fault in a magnetron in a radiotherapy device system according to an aspect of the disclosure;
Figure 4 illustrates a method of predicting a fault in a radiotherapy device according to an aspect of the disclosure;
Figure 5 illustrates a method of predicting a fault in a radiotherapy device according to an aspect of the disclosure.

### SPECIFIC DESCRIPTION OF CERTAIN EXAMPLE EMBODIMENTS

Radiotherapy devices are an important tool in modern cancer treatment. Radiotherapy devices are large, complex machines, with many moving parts and inter-operating mechanisms. Despite precision engineering and rigorous testing, some component parts of a radiotherapy machines may start to degrade over the lifetime of the machine. This can sometimes lead to sub-optimal operation and even the occasional safety override.

If at any point during treatment a radiotherapy device starts to function outside of its normal operating parameters, a safety override or "interrupt" occurs, whereby the machine stops delivering radiation to ensure patient safety. Such an event is inconvenient, as it adds time to the treatment, and in some cases means the treatment session must finish prematurely. Unplanned equipment downtime can disrupt planned treatment schedules, and may be expensive for the owner, be it due to loss of revenue, servicing and repair costs, or both.

Radiotherapy machines are beginning to be configured to produce and record a large amount of data as they operate; for example radiotherapy machines are configured to provide sensor readings from a variety of different sensors. These sensors produce data which can be stored in a database. Radiotherapy devices may also be configured to allow remote connection, enabling service engineers to access a wealth of information about any connected machine without having to travel to the site where the machine is located. It is expected that, in many cases, machines may be returned to optimal performance without an engineer ever having to physically interact with the machine.

However, there will still be occasions where the fault cannot be fixed remotely, and an engineer must be sent to: inspect the machine; determine the nature of the fault; and perform any maintenance required. If the repair involves replacing a part, further machine downtime is required before the machine can be brought back online.

Currently, in medical imaging there is no data routinely recorded from the magnetron. If the magnetron malfunctions, or another component of the radiotherapy machine malfunctions, a mechanic may manually test components of the machine. This may involve testing the parameters of the magnetron through manually, and temporarily, attaching an oscilloscope to the magnetron. This is done during machine "downtime", i.e. when the machine is not operational. The output parameters and behaviours of the magnetron therefore are only tested or recorded once the radiotherapy machine malfunctions. Unplanned equipment downtime can disrupt planned treatment schedules.

The behaviour of a magnetron over its lifetime is not tracked or recorded. Particularly, conditions a magnetron has been exposed to, or the behaviours of the magnetron, towards the end of its life are not tracked of analysed. There is no known way of identifying magnetrons which are approaching the end of their operational life.

Further, measurements of magnetron output are not taken during routine operation of a radiotherapy device, but are only taken by an onsite service engineer. This will be either during unplanned downtime or planned downtime. Since under normal operation the output of a magnetron is not measured, a magnetron may be operating within a performance range which, whilst acceptable to meeting the performance requirements, is not optimal. Performing outside optimal conditions may lead to a shortening of the life span of the magnetron, or to less efficient or suboptimal performance of the radiotherapy device.

Finally, since measurements of the output of the magnetron can currently only be taken with a service engineer on site, and are not taken during routine operation of the radiotherapy device, there is a lack of data relating to performance of a magnetron in a radiotherapy device over its lifetime. It has to date not been possible to establish patterns relating to the performance of a magnetron. Under the present set up, there is not enough data to determine particular data patterns which are may indicative of a particular fault.

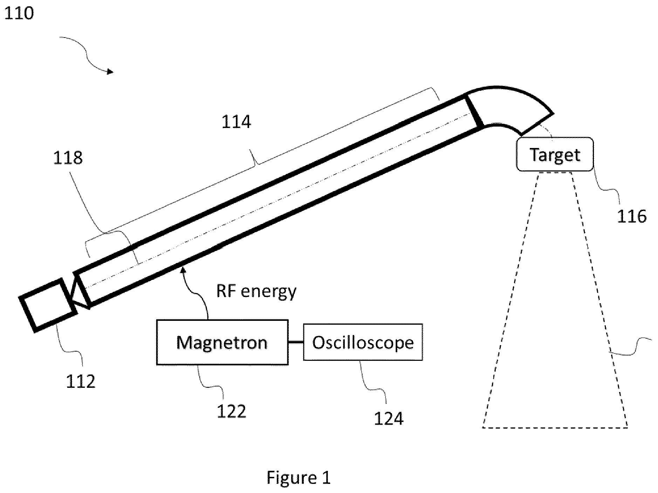

A high-level overview radiotherapy decide according to the disclosure is illustrated in Figure 1. The linear accelerator 110 includes a source of electrons 112, a waveguide 114, and a target 116. Electrons are emitted from the electron gun and accelerated through the waveguide along an acceleration path 118 which is coincident with the centre axis of the waveguide. The electron beam is bent using magnets and strikes the target 116, to produce an x-ray beam 120. The x-ray beam 120 is used to treat a patient.

The source of radiofrequency waves 122, such as a magnetron, produces radiofrequency (RF) waves. The source of radiofrequency waves is coupled to the waveguide, and is configured to pulse radiofrequency waves into the waveguide.

The source of electrons 112 may be an electron gun. The source of electrons is configured to inject electrons into the waveguide 114. The waveguide 114 comprises a plurality of interconnected acceleration cavities (not shown) forming a channel through which the electron beam passes. The injection of electrons into the waveguide 114 is synchronised with the pumping of the radiofrequency waves into the waveguide 114.

The design and operation of the radiofrequency wave source 122, electron source 112 and the waveguide 114 is such that the radiofrequency waves accelerate the electrons to very high energies as they propagate through the waveguide 114 down the acceleration path 118. The waveguide is designed in order that a suitable electric field pattern is produced which accelerates electrons propagating through the waveguide 114.

A magnetron comprises a cathode that also serves as filament. A magnetic field causes electrons emitted by the cathode to spiral outward through a cavity. As the electrons pass the cavity they induce a resonant, RF field in the cavity through the oscillation of charges around the cavity. The RF field can then be extracted with a short antenna attached to one of the spokes.

The device further comprises an oscilloscope 124. The oscilloscope is configured to measure the output of the magnetron. The oscilloscope 124 may be a micro-oscilloscope. The oscilloscope 124 is configured to measure current and voltage of the magnetron anode. The oscilloscope is configured to send signals indicative of the output of the magnetron to the linear accelerator (linac) network. The linac network is the computer network in the linac, which connects the controllers and sensors of the linac. As is explained below, the oscilloscope is configured to send the measurements of the output of the magnetron to a central server (cloud solution). This is done through sending the measurements to the linac network. In another embodiment the oscilloscope is connected to a network and transmits the data to the cloud.

A processor receives signals from the oscilloscope and sends data to the central server. The data is based on the signals received from the oscilloscope. The processor sends data indicative of the output of the magnetron to the central server.

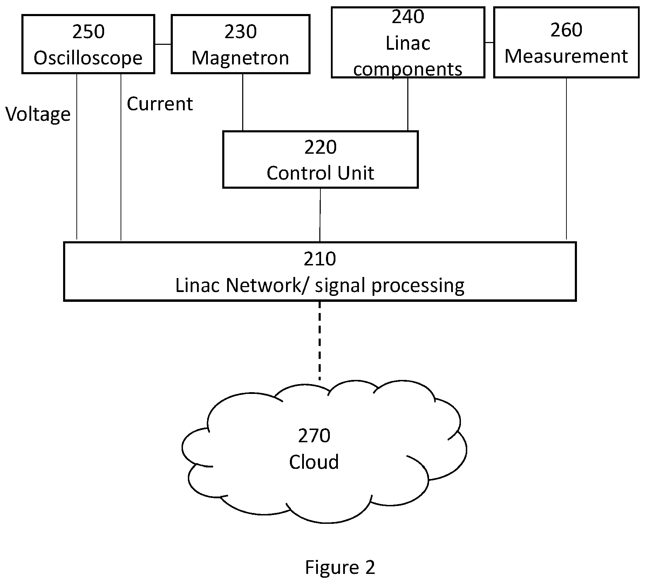

Figure 2 illustrates a block diagram of a linear accelerator network. The connections between components are wired electrical connections. In some embodiments the connections may be wireless.

The linear accelerator has a control until 220 which is configured to control the linear accelerator to deliver a treatment plan to a patient. The control until 220 controls the magnetron 230 to output a required amount of rf energy to the waveguide. The control unit 220 also controls the other components of the linear accelerator 240. Controlling the other components 240 can include: controlling the electron gun to feed electrons to the waveguide; controlling the gantry to rotate according to the treatment plan to provide the angle which radiation is delivered to the patient; and controlling a collimator, such as a multi leaf collimator, MLC, to collimate the beam according to the treatment plan.

The control unit 220 sends information on the linac network 210. The linac network 210 may send the treatment plan to the control unit 220 ahead of the treatment delivery.

The magnetron 240 is controlled by the control unit to 220 to provide rf energy to the waveguide of the linac. The output of the magnetron 230 is measured by oscilloscope 250. The oscilloscope sends the measurements to the linac network. The measurements may be sent through wired or wireless connections to the linac network. The oscilloscope may be a headless oscilloscope which sends the data to an ARM based micro-computer. This micro-computer is connected to the linac network.

The control 220 also controls other components 240 of the linac, for example the electron gun, the gantry and/or the multi-leaf collimator. Outputs of each of these components are sent to the linac network 210. The outpours are measured by different measurement devices. The output of the beam itself may also be measured, and the measurement sent to the linac network 240. The measurements of the beam and of the outputs of the components of the linac are sent to the linac network as electrical signals through an electrical connection, or are sent wirelessly.

The linac network 210 has a wireless connection to the internet. The linac network can send information to a central server such as Axeda or Thingworx. The data can then be automatically analyzed and stored.

The data may be sent - that is transmitted - over a wireless network such as an internet or intranet, WLAN connection or a peer-to-peer connection. Data from the linac network can be sent to a central server storage. The central server, such as a cloud solution, can be accessed remotely, for example from a computer at a central location which is also connected to the internet.

That is, the linac network is configured to send data to the central server, and data on the central server can be accessed from a device which is located at a location remote from the linear accelerator.

The data which is transmitted to the cloud in the system of Figure 2 includes:
- The treatment plan (including the dose of radiation, the shape of the radiation beam, the angles at which the radiation is delivered, the timing of pulses of radiation and any other information relating to the delivery of radiation)
- The output of the magnetron
- The output of other components of the linear accelerator.

The data can be used in the following ways.

### Predictive maintenance

The present methods involve evaluating the condition and/or performance of a magnetron during its operation in order to identify and determine, preferably remotely, whether the magnetron is nearing the end of its operational life and thus whether the magnetron should be replaced or repaired. In particular, the present application relates to taking measurements of the output of a magnetron in a radiotherapy device, and sending the data to a cloud. Analysis of the measurement of the magnetron can be performed and, if the magnetron is not performing optimally, or if the magnetron requires replacement or repair, a service engineer can be sent to the site.

Further, the measurements from the magnetron can be analysed along with other data from the radiotherapy device, such as treatment plans which the device has delivered, to determine patterns in the data. These patterns may be used in predictive maintenance of magnetrons. For example, certain outputs of the magnetron may be indicative that the magnetron may soon need service or repair. Additionally or alternatively, certain types of treatment plans in combination with outputs of the magnetron may be indicative that the magnetron may soon need service or repair.

Such techniques are advantageous as they allow a manufacturer or maintenance service provider to attend the machine knowing what will be required to fix the machine prior to arrival. The disclosed techniques allow the operation of the magnetron to be monitored, and hence magnetrons which are approaching the end of their operational life but which are still operating within required safety parameters can be identified. This in turn allows, for example, repair and/or replacement of the electron gun to be scheduled for the next convenient service point.

The disclosed methods help to reduce machine downtime and thereby minimise disruption to the machine's normal operation. The disclosed techniques can also be used to more effectively plan machine downtime for times which are more convenient or cost-effective for the owner of the equipment and/or the patients. Data collected from the magnetron can also be used in the future for trending and to learn how a magnetron behaves during its lifespan. Data can also be used in further research and development of magnetrons for radiotherapy devices.

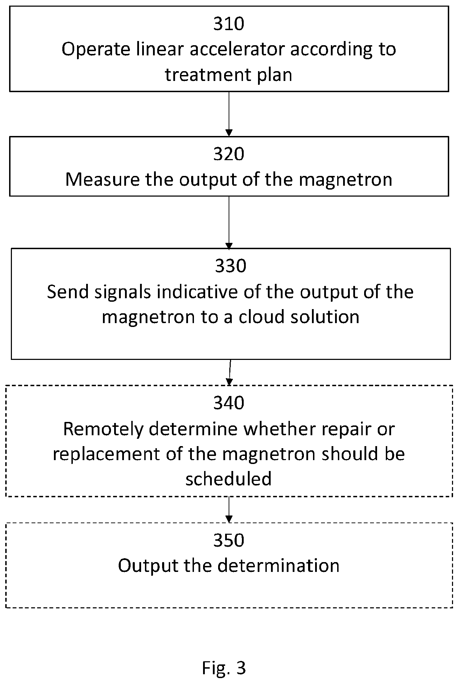

Figure 3 shows a method of obtaining information on the output of a magnetron in a radiotherapy device during routine operation of the magnetron. At step 310, a linear accelerator of a radiotherapy device is operated according to a treatment plan. At the 320, the output of a magnetron in the linear accelerator is measured using an integrated oscilloscope. Signals indicative of the output of the magnetron are sent to a cloud solution at step 330. The signals indicative of the output are sent to the cloud via the internet.

Steps 340 and 350 are not performed on site by the linear accelerator or the associated network, but remotely on the central server.

The data is then used to determine whether repair or replacement of the magnetron should be scheduled 340. For example, if the magnetron is malfunctioning, or if the signals received indicate that the magnetron is operating outside of its optimal parameters, the process determines that a repair or replacement of the magnetron should be scheduled.

At step 350, the determination is output. This may be in the form of an automated message being sent to a service engineer. When this information is analysed it can trigger automatic messages for service engineers and managers to perform a service intervention on the affected machine/s.

These automated messages include diagnostics flowcharts and instructions to aid the engineer during fault finding in order to reduce mean time to repair.

If an incorrect configuration of the magnetron related settings is detected, the engineer receives an automated message which prompts him to adjust these parameters. These parameters can be adjusted remotely.

If the magnetron is showing signs of deterioration, the engineer receives an automated message which prompts him to replace the magnetron.

Both of these activities can be scheduled outside clinical hours to minimise clinical downtime. Scheduling activities outside of clinical hours can also reduce travel time, as well as the mean time to repair.

It would also be possible to automatically create a customer lifecycle management case in parallel to the automated message so that a task it's created and the engineer needs to take action.

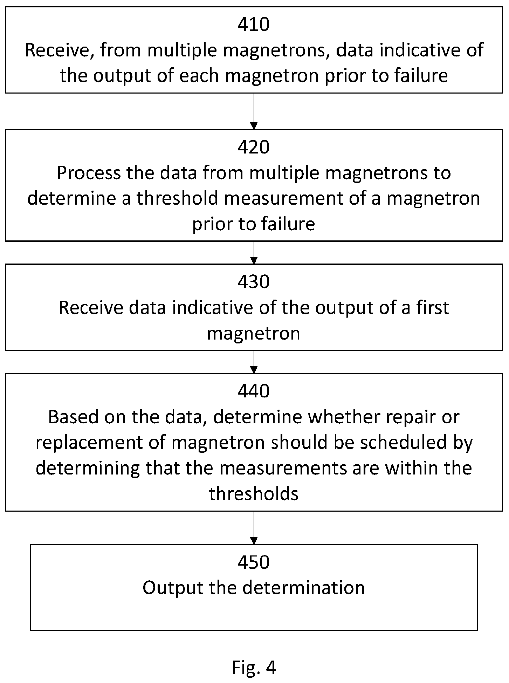

Figure 4 shows a flow diagram of analysing data at a central server to determine whether a magnetron has an increased likelihood of failure.

The method includes: receiving 410, from multiple magnetrons, signals comprising data indicative of the output of each magnetron prior to failure of that magnetron.

The output of the magnetron measured can include a voltage trace and a current trace.

The method further includes processing the data to determine a threshold measurement of a magnetron prior to failure.

Processing the data could take the following form:
- For predictive failure, the pulse widths of the magnetron anode current and voltage are analysed, as well as detecting whether or not there is ripple (noise) in unwanted areas of these signals.
- For incorrect setup, the amplitude of the signals is measured and determine if all inputs have been set correctly.

The server then receives data from a first magnetron, the data indicative of the output of the first magnetron. Based on the data, the server determines that the measurements are within the threshold measurement to determine whether repair or replacement of the electron gun should be scheduled. Finally, the determination is output. The output may be in the form of an automated message.

There is also provided a computer readable medium configured to perform the method of Figure 4.

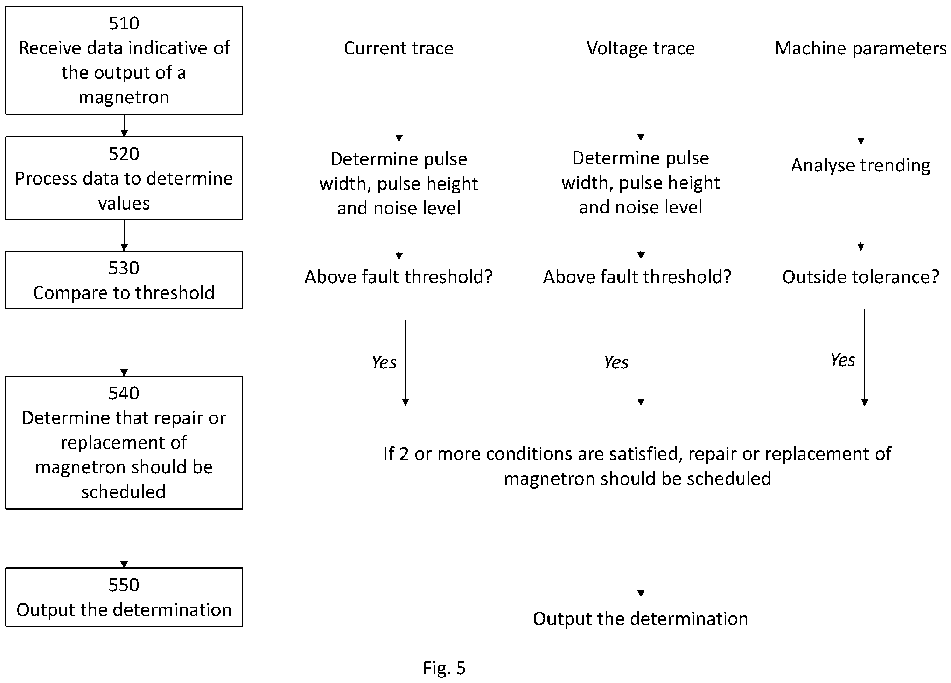

Figure 5 discloses a decision tree for analysing data to determine whether a magnetron has an increased likelihood of failure.

At step 510 at least one of the following measurements are stored in the cloud:
- a trace of the magnetron current;
- a trace of the magnetron voltage;
- other machine parameters, for example data from other components of the linear accelerator.

At step 520, the data is processed to determine values. This includes:
- for the magnetron current trace, determining a value of: the pulse width, height and noise level
- for the magnetron voltage trace, determining a value of: the pulse width, height and noise level
- for the machine parameters, analysing trending of the data items.

At step 530, the data is compared to a threshold. At step 540, a determination is made as to whether the repair or replacement the magnetron should be scheduled based on the comparison.

If any two or more of the following conditions are satisfied, it is determined that the magnetron is nearing the end of its life cycle the repair or replacement the magnetron should be scheduled;
- the value determined for the current trace is greater than the fault threshold;
- the value determined for the voltage trace is greater than the fault threshold;
- the machine parameters are outside of the tolerance.

The determination is then output. This may be in the form of an automated message being sent to a service engineer. When this information is analysed it can trigger automatic messages for service engineers and managers to perform a service intervention on the affected machines. These automated messages include diagnostics flowcharts and instructions to aid the engineer during fault finding in order to reduce mean time to repair.

One of the advantages is that it will be possible to automatically analyse all of the machines connected to the cloud solution and reduce number of failures due to human error as well as number of unplanned service activities. The data from magnetrons will provide a new source of information which can help for future development of linear accelerators. In addition to the previous advantages and as a result of removal of human factor, the life of magnetrons will be increased because they will always be operating within specification limits.

Features of the above aspects can be combined in any suitable manner. It will be understood that the above description is of specific embodiments by way of aspect only and that many modifications and alterations will be within the skilled person's reach and are intended to be covered by the scope of the appended claims.

It remains, that the scope of the invention is defined by the claims.

## Claims

1. A method of determining whether repair or replacement of a magnetron (122, 230) of a linear accelerator should be scheduled, the method comprising:
receiving data indicative of the output of the magnetron in operation;
processing the data to determine values of the output of the magnetron;
comparing the determined values to a threshold;
based on the comparison, determining whether repair or replacement of a magnetron should be scheduled;
outputting the determination whether repair of replacement of the magnetron should be scheduled.

2. The method of claim 1, wherein the threshold is determined by a method comprising:
receiving, from a plurality of magnetrons in operation, data indicative of the output of each magnetron of the plurality of magnetrons prior to failure; processing the data from each magnetron of the plurality of magnetrons to determine the threshold indicative of magnetron failure.

3. The method of claim 1 or 2, wherein the data is indicative of the current trace of the magnetron output; and wherein processing the data to determine values comprises determining the pulse width, pulse height and noise level of the current trace.

4. The method of any of claims 1 to 3, wherein the data is indicative of the voltage trace of the magnetron output; and wherein processing the data to determine values comprises determining the pulse width, pulse height and noise level of the voltage trace.

5. The method of any preceding claim, wherein the data indicative of the output of a magnetron comprises one or more machine parameters, and wherein processing the data comprises analysing trending of the machine parameters.

6. The method of any preceding claim wherein: receiving data indicative of the output of a magnetron comprises receiving three sets of data; wherein processing the data to determine values of the output of the magnetron comprises determining a value for each set of data; comparing each of the determined values to a threshold comprises comparing each determined value to a respective threshold to determine if a condition is met; if two or more of the conditions are met, determining that repair or replacement of a magnetron should be scheduled.

7. A computer readable medium configured to perform the method of any preceding claim.

8. A system comprising:
a computer readable medium according to claim 7;
a central server; and
a linear accelerator system comprising:
a waveguide for accelerating electrons along an acceleration path;
a magnetron configured to supply a radiofrequency electromagnetic field to the waveguide;
an oscilloscope connected to the magnetron and configured to provide signals indicative of the magnetron output;
a processor configured to receive signals from the oscilloscope and to send data to the central server.

9. The system of claim 8, wherein the linear accelerator system further comprises a control unit for controlling operation of the linear accelerator, wherein the control unit is configured to transmit data relating to operation of the linear accelerator to the central server.

10. The system of 9, wherein the control unit is configured to control operation of the linear accelerator in accordance with a treatment plan, and wherein the control unit is configured to transmit information relating to the treatment plan to the central server.

11. The system of claim 9 or 10, wherein the control unit is configured to transmit information comprising at least one of: length and frequency of electron pulses delivered by the linear accelerator; total time of operation of the linear accelerator; total number of pulses delivered by the linear accelerator.

12. The system of any of claims 8 to 11, wherein the signals indicative of the magnetron output comprise signals indicative of the magnetron voltage and current pulses.

13. The system of any of claims 8 to 12, further comprising a sensor configured to provide signals indicative of the output of at least one other component of the linear accelerator, wherein the processor is further configured to send the signals indicative of the output of the at least one other component of the linear accelerator to the central server.

14. The system of any of claims 8 to 13, wherein the processor is configured to send data to the central server via an internet, intranet, WLAN or peer-to-peer connection.

15. The system of any of claims 8 to 14, wherein the data is sent to the central server to be accessed at a location remote from the linear accelerator.

## Patentansprüche

1. Verfahren zum Bestimmen, ob Reparatur oder Austausch eines Magnetrons (122, 230) eines Linearbeschleunigers geplant werden sollte, wobei das Verfahren Folgendes umfasst:
Empfangen von Daten, die die Ausgabe des Magnetrons im Betrieb angeben;
Verarbeiten der Daten, um Werte der Ausgabe des Magnetrons zu bestimmen;
Vergleichen der bestimmten Werte mit einer Schwelle;
Bestimmen, ob Reparatur oder Austausch eines Magnetrons geplant werden sollte, basierend auf dem Vergleich;
Ausgeben der Bestimmung, ob Reparatur oder Austausch des Magnetrons geplant werden sollte.

2. Verfahren nach Anspruch 1, wobei die Schwelle durch ein Verfahren bestimmt wird, das Folgendes umfasst:
Empfangen von Daten, die die Ausgabe jedes Magnetrons der Vielzahl der Magnetrone vor dem Ausfall angeben, von einer Vielzahl der Magnetrone im Betrieb; Verarbeiten der Daten von jedem Magnetron der Vielzahl der Magnetrone, um die Schwelle zu bestimmen, die Ausfall des Magnetrons angibt.

3. Verfahren nach Anspruch 1 oder 2, wobei die Daten die Stromkurve der Magnetronausgabe angeben; und wobei Verarbeiten der Daten zum Bestimmen von Werten Bestimmen der Pulsbreite, der Pulshöhe und des Rauschpegels der Stromkurve umfasst.

4. Verfahren nach einem der Ansprüche 1 bis 3, wobei die Daten die Spannungskurve der Magnetronausgabe angeben; und wobei Verarbeiten der Daten zum Bestimmen von Werten Bestimmen der Pulsbreite, der Pulshöhe und des Rauschpegels der Spannungskurve umfasst.

5. Verfahren nach einem der vorhergehenden Ansprüche, wobei die Daten, die die Ausgabe eines Magnetrons angeben, einen oder mehrere Maschinenparameter umfassen, und wobei Verarbeiten der Daten Analysieren von Trendbildung der Maschinenparameter umfasst.

6. Verfahren nach einem der vorhergehenden Ansprüche, wobei Empfangen von Daten, die die Ausgabe eines Magnetrons angeben, Empfangen von drei Datensätzen umfasst; wobei Verarbeiten der Daten, um Werte der Ausgabe des Magnetrons zu bestimmen, Bestimmen eines Werts für jeden Datensatz umfasst; Vergleichen jedes der bestimmten Werte mit einer Schwelle Vergleichen jedes bestimmten Werts mit einer jeweiligen Schwelle umfasst, um zu bestimmen, ob eine Bedingung erfüllt ist; wenn zwei oder mehr der Bedingungen erfüllt sind, Bestimmen, dass Reparatur oder Austausch eines Magnetrons geplant werden sollte.

7. Computerlesbares Medium, das dazu ausgelegt ist, das Verfahren nach einem der vorhergehenden Ansprüche durchzuführen.

8. System, umfassend:
ein computerlesbares Medium nach Anspruch 7;
einen zentralen Server; und
ein Linearbeschleunigersystem, umfassend:
einen Wellenleiter zum Beschleunigen von Elektronen entlang eines Beschleunigungswegs;
ein Magnetron, das dazu ausgelegt ist, dem Wellenleiter ein elektromagnetisches Hochfrequenzfeld zuzuführen;
ein Oszilloskop, das mit dem Magnetron verbunden ist und dazu ausgelegt ist, Signale bereitzustellen, die die Magnetronausgabe angeben;
einen Prozessor, der dazu ausgelegt ist, Signale von dem Oszilloskop zu empfangen und Daten an den zentralen Server zu senden.

9. System nach Anspruch 8, wobei das Linearbeschleunigersystem ferner eine Steuereinheit zum Steuern des Betriebs des Linearbeschleunigers umfasst, wobei die Steuereinheit dazu ausgelegt ist, Daten, die mit dem Betrieb des Linearbeschleunigers in Beziehung stehen, an den zentralen Server zu übertragen.

10. System nach Anspruch 9, wobei die Steuereinheit dazu ausgelegt ist, den Betrieb des Linearbeschleunigers gemäß einem Behandlungsplan zu steuern, und wobei die Steuereinheit dazu ausgelegt ist, Informationen, die mit dem Behandlungsplan an den zentralen Server in Beziehung stehen, zu übertragen.

11. System nach Anspruch 9 oder 10, wobei die Steuereinheit dazu ausgelegt ist, Informationen zu übertragen, die mindestens eines von Länge und Frequenz von Elektronenpulsen, die durch den Linearbeschleuniger abgegeben werden; Gesamtzeit des Betriebs des Linearbeschleunigers; Gesamtanzahl von Pulsen, die durch den Linearbeschleuniger abgegeben werden, umfassen.

12. System nach einem der Ansprüche 8 bis 11, wobei die Signale, die die Magnetronausgabe angeben, Signale umfassen, die die Spannungs- und Strompulse des Magnetrons angeben.

13. System nach einem der Ansprüche 8 bis 12, ferner umfassend einen Sensor, der dazu ausgelegt ist, Signale bereitzustellen, die die Ausgabe mindestens einer anderen Komponente des Linearbeschleunigers angeben, wobei der Prozessor ferner dazu ausgelegt ist, die Signale, die die Ausgabe der mindestens einen anderen Komponente des Linearbeschleunigers angeben, an den zentralen Server zu senden.

14. System nach einem der Ansprüche 8 bis 13, wobei der Prozessor dazu ausgelegt ist, Daten über eine Internet-, Intranet-, WLAN- oder Peer-to-Peer-Verbindung an den zentralen Server zu senden.

15. System nach einem der Ansprüche 8 bis 14, wobei die Daten an den zentralen Server gesendet werden, damit an einem von dem Linearbeschleuniger entfernten Ort darauf zugegriffen werden kann.

## Revendications

1. Procédé permettant de déterminer si la réparation ou le remplacement d'un magnétron (122, 230) d'un accélérateur linéaire doit être programmé, le procédé comprenant :
la réception de données indicatives de la sortie du magnétron en fonctionnement ;
le traitement des données pour déterminer les valeurs de la sortie du magnétron ;
la comparaison des valeurs déterminées à un seuil,
sur la base de la comparaison, la détermination si la réparation ou le remplacement d'un magnétron devrait être planifié ;
la production de la détermination si la réparation du remplacement du magnétron doit être planifiée.

2. Procédé selon la revendication 1, le seuil étant déterminé par un procédé comprenant :
la réception, à partir d'une pluralité de magnétrons en fonctionnement, de données indicatives de la sortie de chaque magnétron de la pluralité de magnétrons avant la défaillance ; le traitement des données provenant de chaque magnétron de la pluralité de magnétrons pour déterminer le seuil indiquant la défaillance du magnétron.

3. Procédé selon la revendication 1 ou 2, les données étant indicatives du tracé courant de la sortie magnétron ; et le traitement des données pour déterminer des valeurs comprenant la détermination de la largeur d'impulsion, de la hauteur d'impulsion et du niveau de bruit du tracé courant.

4. Procédé selon l'une quelconque des revendications 1 à 3, les données étant indicatives de la trace de tension de la sortie magnétron ; et le traitement des données pour déterminer des valeurs comprenant la détermination de la largeur d'impulsion, de la hauteur d'impulsion et du niveau de bruit de la trace de tension.

5. Procédé selon l'une quelconque des revendications précédentes, les données indicatives de la sortie d'un magnétron comprenant un ou plusieurs paramètres de machine, et le traitement des données comprenant l'analyse des tendances des paramètres de machine.

6. Procédé selon l'une quelconque des revendications précédentes, la réception de données indicatives de la sortie d'un magnétron comprenant la réception de trois ensembles de données ; le traitement des données pour déterminer des valeurs de la sortie du magnétron comprenant la détermination d'une valeur pour chaque ensemble de données ; la comparaison de chacune des valeurs déterminées à un seuil comprenant la comparaison de chaque valeur déterminée à un seuil respectif pour déterminer si une condition est satisfaite ; si au moins deux des conditions sont remplies, la détermination que la réparation ou le remplacement d'un magnétron doit être planifié.

7. Support lisible par ordinateur, configuré pour réaliser le procédé selon n'importe quelle revendication précédente.

8. Système comprenant :
un support lisible par ordinateur selon la revendication 7 ;
un serveur central ; et
un système d'accélérateur linéaire comprenant :
un guide d'onde pour l'accélération des électrons le long d'un chemin d'accélération ;
un magnétron configuré pour fournir un champ électromagnétique radiofréquence au guide d'onde,
un oscilloscope connecté au magnétron et configuré pour fournir des signaux indicatifs de la sortie du magnétron ;
un processeur configuré pour recevoir des signaux en provenance de l'oscilloscope et pour envoyer des données au serveur central.

9. Système selon la revendication 8, le système d'accélérateur linéaire comprenant en outre une unité de commande pour commander le fonctionnement de l'accélérateur linéaire, l'unité de commande étant configurée pour transmettre des données concernant le fonctionnement de l'accélérateur linéaire au serveur central.

10. Système selon la revendication 9, l'unité de commande étant configurée pour commander le fonctionnement de l'accélérateur linéaire conformément à un plan de traitement, et l'unité de commande étant configurée pour transmettre au serveur central des informations relatives au plan de traitement.

11. Système selon la revendication 9 ou 10, l'unité de commande étant configurée pour transmettre des informations comprenant au moins l'un parmi : la longueur et la fréquence des impulsions d'électrons délivrées par l'accélérateur linéaire ; le temps total de fonctionnement de l'accélérateur linéaire ; le nombre total d'impulsions délivrées par l'accélérateur linéaire.

12. Système selon l'une quelconque des revendications 8 à 11, les signaux indicatifs de la sortie de magnétron comprenant des signaux indicatifs de la tension de magnétron et des impulsions de courant.

13. Système selon l'une quelconque des revendications 8 à 12, comprenant en outre un capteur configuré pour fournir des signaux indicatifs de la sortie d'au moins un autre composant de l'accélérateur linéaire, le processeur étant en outre configuré pour envoyer les signaux indicatifs de la sortie de l'au moins un autre composant de l'accélérateur linéaire au serveur central.

14. Système selon l'une quelconque des revendications 8 à 13, le processeur étant configuré pour envoyer des données au serveur central via une connexion Internet, intranet, WLAN ou poste à poste.

15. Système selon l'une quelconque des revendications 8 à 14, les données étant envoyées au serveur central pour y accéder à un emplacement éloigné de l'accélérateur linéaire.
